# EUROPEAN PATENT APPLICATION

(11) **EP 3 241 524 A1**
(43) Date of publication of application: **08.11.2017**
(21) Application number: 15874796.4
(22) Date of filing: 24.06.2015
(51) Int. Cl.: A61F 2/02, A61L 27/34, A61L 27/56

(54) **SOLID-CORE NERVE SCAFFOLD HAVING BUILT-IN DEGRADABLE METAL WIRE**

(30) Priority: 29.12.2014 CN 201420851211 U
(71) Applicant: Dongguan Dianfu Product Design Co., Ltd., Dongguan, Guangdong 523000 (CN)
(72) Inventor: LI, Yangde, Dongguan Guangdong 523000 (CN)
(74) Representative: Hryszkiewicz, Danuta
(86) International application number: PCT/CN2015/082154
(87) International publication number: WO 2016/107106

(57) **Abstract**

A solid-core nerve scaffold having a built-in degradable metal wire, comprising a scaffold membrane (1) and the degradable metal wire (2) wrapped by the scaffold membrane (1). The scaffold membrane (1) is made of a biodegradable material and provides a three-dimensional gap structure. The degradable metal wire (2) is distributed in the transverse and longitudinal directions of the nerve scaffold. The nerve scaffold not only provides the three-dimensional structure required for neuroregeneration, but also is capable of promoting and guiding neurogenesis, has great mechanical properties, and is biodegradable.

## Description

The invention relates to the field of medical instruments, and more particularly to a solid nerve scaffold comprising a build-in metal wire.

Peripheral nerve injury or rupture caused by various trauma often leads to the decline or loss of the sense and motor function of the wounded. Severe peripheral nerve injuries often result in paralysis or permanent loss of labor. The incidence of peripheral nerve injury is much high in China. According to statistics, in patients with trauma, limb nerve injuries accounted for about 10% of the total number of injuries, fractures resulting from firearms injuries contain about 60% of the combined nerve injury. Therefore, it is necessary to find an ideal treatment for peripheral nerve injuries. For the peripheral nerve fracture, if the fracture gap is large, the nerve defect requires bridging so as to reconstruct the broken peripheral nerve.

Currently, the commonly used grafts include autografts and artificial nerve scaffolds. The autografts have limited sources and often causes donor sequelae. The artificial nerve scaffolds are often made of non-degradable catheter materials which may cause the risks such as nerve compression and secondary surgery. So, in recent years, the development and research mainly focus on the biodegradable materials. Currently, the United States has approved a variety of brands such as NeuraGen, SaluBridge, Neurolac and other nerve conduits for clinical use.

The nerve scaffolds made of biodegradable materials can bridge the defective nerve after being implanted in human body, however, these products provide only a bridge or passage for the growth of regenerating axons, do not promote the growth of nerve.

In view of the above-described problems, it is one objective of the invention to provide a solid nerve scaffold comprising a degradable metal wire. The nerve scaffold has a three-dimensional structure that is favorable to nerve regeneration, can promote and guide nerve growth, appropriately matches the implantation environment in terms of mechanical properties, and is biodegradable.

To achieve the above objective, in accordance with one embodiment of the invention, there is provided a nerve scaffold, comprising a membrane material; and a degradable metal wire. The degradable metal wire is enclosed in the membrane material; the membrane material is biodegradable material, and comprises three-dimensional pore structure; and the degradable metal wire is horizontally and vertically distributed in the nerve scaffold.

In a class of this embodiment, the nerve scaffold is cylindrical, strip, or flaky in shape.

In a class of this embodiment, the nerve scaffold is between 4 and 30 mm in length.

In a class of this embodiment, the three-dimensional pore structure accounts for 10-65% of the nerve scaffold.

In a class of this embodiment, the degradable metal wire is magnesium, zinc, calcium, iron, or an alloy thereof.

In a class of this embodiment, the membrane material is biodegradable material selected from the group consisting of collagen, polylactic acid, chitosan, hydroxyapatite, or a composite thereof.

In a class of this embodiment, the membrane material is originated from an animal blood vessel, lymph vessel, nerve canal, intestine, or muscle, of which the cells are extracted chemically.

Advantages of the nerve scaffold according to embodiments of the invention are summarized as follows: the nerve scaffold has a three-dimensional structure favorable to nerve regeneration, can promote and guide the nerve growth, has good mechanical property, is biodegradable, and is significant for the neural restoration.
FIG. 1 is a schematic diagram of a solid nerve scaffold comprising a build-in degradable metal wire in accordance with one embodiment of the invention; and
FIG. 2 is a schematic diagram of a solid nerve scaffold comprising a build-in degradable metal wire in accordance with another embodiment of the invention.

For further illustrating the invention, experiments detailing a solid nerve scaffold comprising a build-in degradable metal wire are described hereinbelow combined with the drawings. It should be noted that the following examples are intended to describe and not to limit the invention.

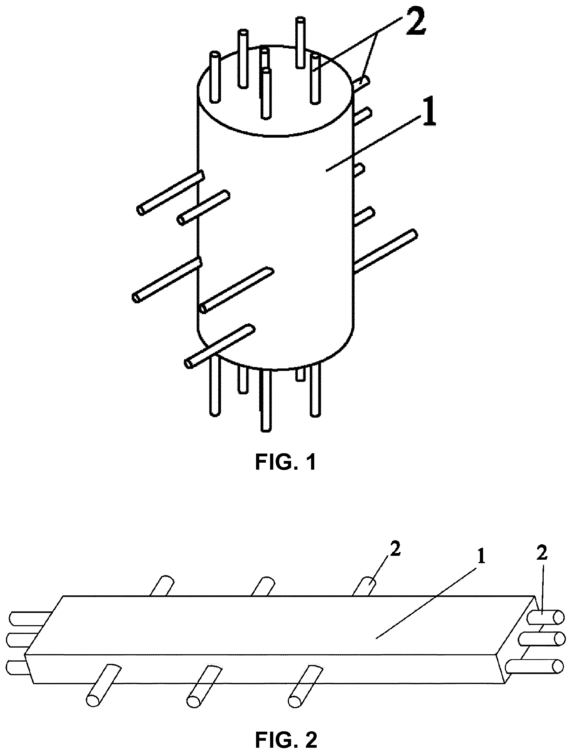

As shown in FIG. 1, a solid nerve scaffold comprising a build-in degradable metal wire comprises a membrane material 1 and a degradable metal wire 2 which is enclosed in the membrane material 1. The degradable metal wire 2 is horizontally and vertically distributed in the nerve scaffold. The nerve scaffold is between 4 and 30 mm in length.

The nerve scaffold can be manufacture into desired shape as needed, in general, it is cylindrical, strip, or flaky in shape. FIG. 1 shows a cylindrical scaffold and FIG. 2 shows a flaky scaffold. It should be noted that, the shape of the nerve scaffold of the present disclosure is not limited to the above shape, and the shape of the nerve support is flexible and variable without departing from the basic conception of the invention.

The membrane material is biodegradable material, and comprises three-dimensional pore structure. The membrane material is biodegradable material selected from the group consisting of collagen, polylactic acid, chitosan, hydroxyapatite, or a composite thereof. The membrane material is originated from an animal blood vessel, lymph vessel, nerve canal, intestine, or muscle, of which the cells are extracted chemically.

The membrane material comprises three-dimensional pore structure. The three-dimensional pore structure accounts for 10-65% of the nerve scaffold, and provides space for the nerve growth.

The degradable metal wire 2 is magnesium, zinc, calcium, iron, or an alloy thereof, which can promote and guide the nerve growth.

Unless otherwise indicated, the numerical ranges involved in the invention include the end values. While particular embodiments of the invention have been shown and described, it will be obvious to those skilled in the art that changes and modifications may be made without departing from the invention in its broader aspects, and therefore, the aim in the appended claims is to cover all such changes and modifications as fall within the true spirit and scope of the invention.

## Claims

1. A nerve scaffold, comprising:
a membrane material; and
a degradable metal wire;
wherein
the degradable metal wire is enclosed in the membrane material;
the membrane material is biodegradable material, and comprises three-dimensional pore structure; and
the degradable metal wire is horizontally and vertically distributed in the nerve scaffold.

2. The scaffold of claim 1, **characterized in that** the nerve scaffold is cylindrical, strip, or flaky in shape.

3. The scaffold of claim 1, **characterized in that** the nerve scaffold is between 4 and 30 mm in length.

4. The scaffold of claim 1, **characterized in that** the three-dimensional pore structure accounts for 10-65% of the nerve scaffold.
